# FASCICULE DE BREVET EUROPEEN

(11) **EP 1 604 038 B1**
(45) Date de publication et mention de la délivrance du brevet: **29.11.2006**
(21) Numéro de dépôt: 04717128.5
(22) Date de dépôt: 04.03.2004
(51) Int. Cl.: C12Q 1/68, C12M 1/00

(54) **INSTALLATION AUTOMATISEE POUR LA DETECTION DE LA FIXATION DE BIOMOLECULES SUR UN ECHANTILLON EN TROIS DIMENSIONS**
AUTOMATISCHE VORRICHTUNG ZUM NACHWEIS DER BIOMOLEKÜLEN-FIXIERUNG AUF EINER DREIDIMENSIONALEN PROBE
AUTOMATED SYSTEM FOR DETECTING THE FIXATION OF BIOMOLECULES ON A SAMPLE IN THREE DIMENSIONS

(30) Priorité: 20.03.2003 FR 0303438
(43) Date de publication de la demande: 14.12.2005
(73) Titulaire: Centre National de la Recherche Scientifique, 75794 Paris Cedex 16 (FR); UNIVERSITE JOSEPH FOURIER, F-34000 Saint-Martin D'Heres (FR)
(72) Inventeur: THELU, Jacques, Lucien, F-38920 Crolles (FR)
(74) Mandataire: Grosset-Fournier, Chantal Catherine
(86) Numéro de dépôt international: PCT/FR2004/000510
(87) Numéro de publication internationale: WO 2004/087953

(56) Documents cités:
- WO-A-02/23167
- WO-A-02/092778
- US-A- 5 932 418
- US-A1- 2003 049 862

## Description

La présente invention est relative à une installation automatisée pour la détection de la fixation de molécules d'origine biologique ou biomolécules sur un échantillon en trois dimensions, permettant notamment le repérage de l'expression de gènes ou de protéines par marquage sélectif de ces molécules dans la masse de l'échantillon au moyen de sondes ADN ou ARN, d'anticorps, d'enzymes ou d'une combinaison appropriée de ces marqueurs.

On connaît déjà dans la technique le procédé qui consiste à marquer sélectivement des échantillons, généralement des coupes minces, qui ne permettent pas de ce fait une observation globale de ces échantillons et limitent l'interprétation des résultats obtenus par un marquage de ceux-ci, ces mesures, effectuées en outre manuellement, étant longues, onéreuses et peu reproductibles.

On connaît également le procédé de marquage sélectif d'une structure en trois dimensions pour réaliser une hybridation au sein du volume global d'un échantillon, notamment pour la détection de l'expression de gènes ou encore d'une immuno-réaction dans le cas de la détection de protéines, ce procédé connu sous le terme d'hybridation « in toto » ou « whole mount », étant usuellement mis en oeuvre au moyen d'un automate à bras télécommandé selon les trois dimensions de l'espace.

Mais ces appareils et leurs modalités d'utilisation sont peu pratiques et surtout ne présentent pas une grande sûreté, notamment du fait de l'exécution d'opérations discontinues de remplissage et de vidange successives des tubes ou autre récipients contenant individuellement les échantillons à traiter. De plus, ils sont totalement inadaptés lorsque les solutions utilisées ainsi que les réactifs employés doivent être placés sous une pression suffisante pour pouvoir pénétrer les échantillons à coeur, surtout si ces derniers sont peu perméables comme c'est le cas avec la plupart des biopsies pratiquées sur des tissus vivants.

La présente invention a pour objet une installation automatisée qui évite ces inconvénients grâce à la mise en oeuvre de moyens qui permettent une détection en continu et sous une pression convenable des effets d'un marquage appliqué à un échantillon biologique en trois dimensions, notamment pour obtenir l'expression de gènes ou de protéines.

A cet effet, l'installation considérée se caractérise en ce qu'elle comporte:
- une pluralité de modules en forme de coupelles individuelles de réception d'un ou plusieurs échantillons à traiter, comportant chacune une paroi latérale fermée par un fond perforé pour le passage en continu à l'intérieur de la coupelle de solutions ou réactifs liquides à mettre en contact avec l'échantillon, ces coupelles présentant le même encombrement diamétral et étant logées en superposition verticale dans un tube ou une enveloppe externe analogue de positionnement et de confinement,
- un circuit pour l'écoulement en continu à travers les coupelles dans le tube de confinement, sous pression réglable, de ces solutions, provenant d'autant de réservoirs distincts, ces solutions étant introduites à la demande, ensemble ou séparément dans ce circuit,
- une électrovanne multi-voies, propre à relier séparément chaque réservoir de solutions au circuit,
- une pompe électrique, de préférence à inversion du sens de pompage et de refoulement, apte à faire circuler en continu dans le circuit les quantités de solutions prélevées depuis les réservoirs,
- et un moyen de contrôle et de commande séquentiel et coordonné de la pompe et de l'électrovanne multi-voies.

Le circuit de l'installation peut être un circuit fermé, en boucle, pour le recyclage permanent des solutions ou bien être ouvert, pour l'élimination de ces solutions après au moins un passage à travers les coupelles dans le tube de confinement.

De préférence, les coupelles sont empilées dans le tube de confinement disposé verticalement, l'écoulement des solutions dans ce tube s'effectuant dans le sens ascendant.

Selon une autre caractéristique, l'installation comporte une seconde électrovanne multi-voies, permettant soit la récupération de certaines au moins des solutions pour les faire circuler en boucle dans le circuit avant de les renvoyer dans leurs réservoirs respectifs, soit l'élimination de ces solutions vers un récipient d'évacuation extérieur.

Avantageusement, les coupelles de réception des échantillons sont cylindriques et présentent la même hauteur d'une coupelle à l'autre. En variante cependant, les coupelles peuvent présenter des hauteurs différentes, notamment selon la taille et l'encombrement des échantillons contenus dans ces coupelles.

Selon une caractéristique particulière, les coupelles de réception des échantillons, empilées dans le tube de confinement, sont immobilisées entre un fond inférieur fixe, fermant ce tube, et un fond supérieur mobile, dont le déplacement à l'intérieur du tube pour s'appliquer sur l'empilement des coupelles, est commandé par un piston d'actionnement extérieur à ce tube, plongeant dans ce dernier et comportant un canal axial pour la circulation des solutions dans le circuit.

De préférence, le fond mobile est équipé d'un racleur étanche, en un matériau élastique approprié, portant contre la surface interne du tube de confinement afin d'éviter la fuite de solutions liquides hors de ce tube.

Avantageusement aussi, la paroi latérale de chaque coupelle est transparente et réalisée en un matériau plastique, notamment en un polymère tel que du polystyrène, du métacrylate de méthyle ou du polycarbonate.

Dans une mode de réalisation préféré, le fond de chaque coupelle est par ailleurs constitué par un tamis à mailles fines, notamment en toile de polyester ou de *« Nylon ».*

Selon une autre caractéristique, le tube de confinement est réalisé en verre ou en une matière plastique transparente.

Selon encore une autre caractéristique, les moyens de contrôle et de commande séquentiels comportent un micro-ordinateur qui détermine la composition, l'ordre de leur écoulement dans le circuit et la durée de mise en contact des solutions ou réactifs avec les échantillons dans les coupelles, ainsi que le débit de l'écoulement de ces solutions.

Dans un mode de réalisation préféré, le tube de confinement est logé dans une enceinte thermostatée et thermorégulée.

Avantageusement, cette enceinte thermostatée comporte une réglette métallique, collée ou autrement appliquée contre une génératrice du tube de confinement, cette réglette étant maintenue en contact avec un bloc de métal massif, contre lequel est rapporté un élément propre à réaliser un chauffage contrôlé de ce bloc.

De préférence également, le circuit comporte un serpentin scellé dans le bloc métallique, placé en série dans ce circuit en amont du tube de confinement, de sorte que les solutions ou réactifs qui traversent les coupelles empilées soient portées à la température régulée de ce bloc métallique. Notamment, le serpentin peut être réalisé en acier inoxydable et le bloc métallique en aluminium.

Utilement, le tube de confinement et la réglette métallique sont entourés par un capot de protection, de préférence en matière plastique.

Grâce à ces diverses dispositions, selon le cas mises en oeuvre ensemble ou séparément, l'invention permet de soumettre les échantillons en trois dimensions placés dans les modules formés par les coupelles superposées à l'intérieur du tube de confinement, à un flux continu de solutions ou de réactifs, évitant toute nécessité de renouvellement intermittent de ceux-ci, notamment avec des cycles successifs de remplissage et de vidange comme dans les réalisations antérieures.

L'installation proposée permet au contraire une automatisation complète du processus, grâce à la mise en oeuvre de la pompe électrique et de l'électrovanne multi-voies, ainsi qu'au pilotage coordonné de ces appareils par le micro-ordinateur.

Ce flux continu des solutions réactives a notamment pour effet d'augmenter l'efficacité de leur transfert au coeur même des échantillons contenus dans les coupelles, sans épuisement ni stagnation de ces réactifs au contact de ces échantillons. Ceci évite que leur dépôt soit superficiel et apporte une efficacité accrue de leur pénétration au coeur de l'échantillon avec des étapes globalement plus courtes.

L'installation est de plus étanche, ce qui présente un réel avantage dans le cas de l'usage d'échantillons dont la nature exige qu'ils soient confinés. Par ailleurs, la pompe électrique peut être utilisée à des pressions variables, notamment supérieure à la pression atmosphérique, pour accroître le transfert des solutions ou réactifs au coeur de ces échantillons, même si ces derniers présentent une certaine imperméabilité, due par exemple à une différenciation cellulaire importante.

De même, grâce à l'usage d'une relativement haute pression dans le circuit, il est possible d'analyser en profondeur certains échantillons présentant une faible pénétrabilité vis-à-vis des réactifs utilisés, notamment tels que des biopsies.

L'installation présente encore l'avantage que chaque échantillon peut être traité dans celle-ci de manière sensiblement identique et parfaitement reproductible, l'expérience montrant que dans un flux permanent de solutions ou de réactifs, les interférences réciproques des conditions de traitement d'un échantillon à l'autre dans des coupelles successives sont compensées par la dynamique de ce flux qui renouvelle et homogénéise constamment l'environnement de ces échantillons et n'affecte pas les résultats des mesures effectuées sur chacun d'eux.

L'installation selon l'invention autorise le recyclage en continu et de préférence dans ce cas en boucle fermée des réactifs liquides les plus coûteux tels que les sondes ADN ou ARN, ou les anticorps, ces réactifs pouvant être ainsi recyclés et utilisés de façon plus diluée que dans le cas d'un procédé statique, les molécules ayant une affinité particulière étant en outre piégées uniquement dans la zone spécifique de l'échantillon.

Cette installation est modulable à volonté et peut mettre en jeu un nombre d'échantillons variables, les dimensions du système étant aisément adaptées au nombre des coupelles, notamment à l'aide du piston mobile et du racleur étanche qu'il comporte.

Elle est enfin conçue pour procurer une thermorégulation précise des conditions dans lesquelles sont réalisées le marquage des échantillons et les mesures effectuées, sans choc thermique sur les solutions et réactifs nouvellement prélevés depuis les réservoirs et introduits dans le circuit, grâce à la circulation de ces solutions dans le serpentin noyé dans le bloc métallique, préalablement à leur passage dans le tube à travers les coupelles empilées.

Ainsi, l'installation permet de modifier au gré de l'utilisateur la température adoptée, la plupart des incubations pouvant être effectuées à la température ambiante contrairement aux étapes d'hybridation qui doivent le plus généralement être réalisées à une température stable plus élevée, de l'ordre de 70°.

La mise en place et le retrait des échantillons et des coupelles dans ou hors de l'installation sont très faciles, pouvant être effectués de façon simple, notamment au moyen d'une pince ou outil similaire par l'extrémité du tube après enlèvement de son fond supérieur mobile, l'usage d'un tube en matière transparente procurant une visibilité satisfaisante.

Lors de la mise en place des coupelles, dont le fond perforé en mailles très fines est difficilement traversé par de l'air, il est possible d'éviter de piéger celui-ci entre deux coupelles successives pendant l'introduction de ces dernières dans le tube, la bulle formée risquant de fausser ultérieurement les mesures. Dans ce but, il suffit d'inverser momentanément le sens d'écoulement des solutions par la pompe afin de compenser la montée du liquide induite par la mise en place de ces coupelles, ceci avant que l'empilement de ces dernières en nombre adéquat ne soit immobilisé par le piston agissant sur le fond mobile et le racleur d'étanchéité appliqués sur la coupelle supérieure dans cet empilement ; le mouvement de la pompe est ensuite inversé pour revenir au sens de circulation normal des solutions et réactifs dans le tube, notamment du bas vers le haut dans celui-ci.

Pour le retrait des échantillons, il suffit de procéder de manière opposée en retirant d'abord le piston, puis en introduisant par la base du tube une quantité d'air appropriée après avoir désamorcé la liaison entre la pompe et un réservoir quelconque de solution. La bulle d'air qui se forme dans ce cas à la base du tube pousse progressivement les coupelles vers le haut sous l'effet du pompage, permettant de récupérer une à une les coupelles et les échantillons contenus dans celles-ci.

D'autres avantages et caractéristiques de l'installation considérée apparaîtront encore à travers la description qui suit d'un mode de réalisation de celle-ci, explicité en référence aux dessins annexés, ainsi que de plusieurs exemples de protocoles pouvant être exploités avec cette installation, ces protocoles étant donnés seulement à titre indicatif et non limitatif.

Sur les dessins annexés :
- la Figure 1 est une vue en perspective et en coupe axiale partielle, d'une coupelle entrant dans la réalisation de l'installation considérée ;
- la Figure 2 est une vue en perspective éclatée du tube de confinement d'un empilement de coupelles mis en oeuvre dans l'installation ;
- la Figure 3 est un schéma général de l'installation ;
- la Figure 4 est une vue en coupe d'un mode de réalisation également schématique du tube de confinement des coupelles et de l'enceinte thermostatée et thermorégulée qui lui est associée.

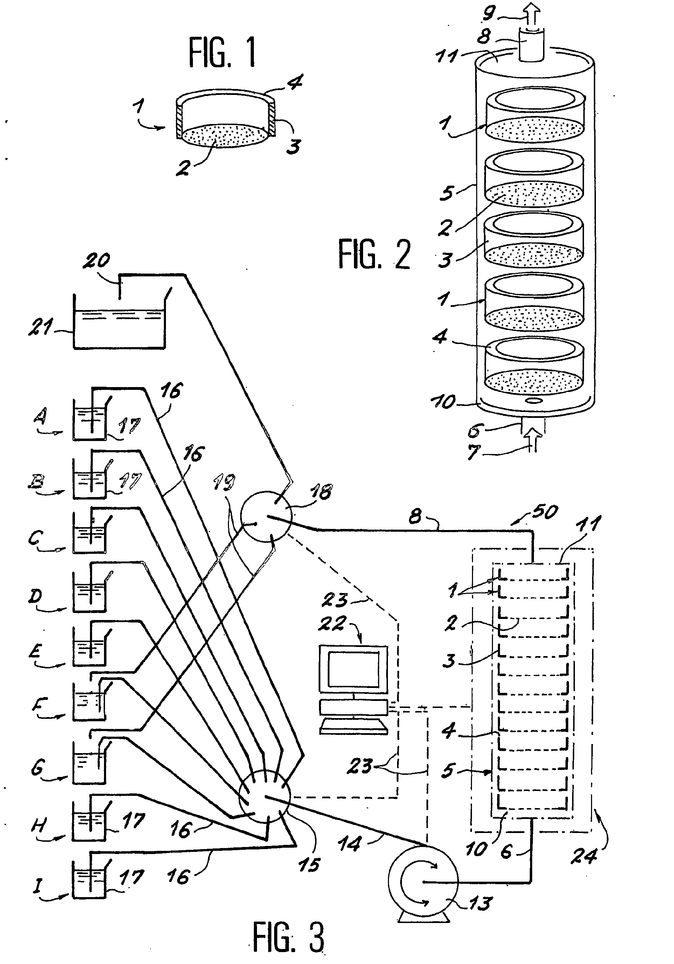

Sur la Figure 1, la coupelle représentée, désignée dans son ensemble sous la référence 1, se compose d'un fond perforé 2, de préférence formé d'une toile de *« Nylon »* (Marque déposée) ou d'un matériau plastique similaire, dont la maille est choisie pour permettre sans perte de charge excessive, le libre écoulement à travers ce fond d'un flux liquide approprié, la toile présentant par ailleurs une tenue mécanique suffisante pour permettre de loger et de supporter à l'intérieur de la coupelle un échantillon en trois dimensions, sur lequel on souhaite réaliser un marquage par une sonde, un anticorps ou une enzyme, ou tout autre élément sélectif adapté.

La coupelle 1 comporte par ailleurs une paroi latérale 3 de forme générale cylindrique dans l'exemple représenté, mais dont la section pourrait être différente, cette paroi étant réunie par tout procédé de liaison convenable au bord périphérique de la toile du fond 2. La coupelle est totalement ouverte à son extrémité 4 opposée à ce fond.

La Figure 2 illustre très schématiquement, en vue éclatée, le montage d'un ensemble de coupelles 1 du genre décrit ci-dessus, ces coupelles étant prévues pour se superposer les unes aux autres continu à l'intérieur d'un tube de confinement 5.

Ce tube 5 est avantageusement réalisé en verre du type « Pyrex » (Marque déposée), ayant une épaisseur appropriée, généralement de l'ordre de 5 mm pour présenter une résistance convenable vis-à-vis de la pression interne des solutions ainsi qu'aux chocs éventuels en cours de manipulation, tout en assurant cependant un bon échange thermique avec un ensemble de régulation en température décrit plus loin, , ou bien être constitué d'un matériau plastique transparent, notamment un polymère du genre polystyrène, métacrylate ou polycarbonate, permettant de voir l'empilement des coupelles 1 à l'intérieur de ce tube.

La section droite de ce dernier est sensiblement égale à celle des coupelles 1, de sorte qu'un écoulement d'une solution liquide appropriée, dont la nature sera précisée plus loin, introduit à la base du tube 5 par un canal de raccordement inférieur 6, notamment selon le sens ascendant représenté par la flèche 7, le tube étant disposé verticalement, traverse intégralement chacune des coupelles 1 de l'empilement pour être finalement repris à la partie supérieure par un canal d'évacuation 8, selon le sens de la flèche 9.

Le tube de confinement 5 est fermé à son extrémité basse par un fond fixe 10, dans lequel débouche le canal de raccordement 6 et comporte à son extrémité supérieure un fond mobile 11 par l'intermédiaire d'un piston 12 (non représenté) sur la Figure 2, mais qui apparaît sur la Figure 4, ce piston venant s'appuyer sur l'empilement des coupelles 1 dans le tube 5 en bloquant chacune d'elles sur celles situées en dessous.

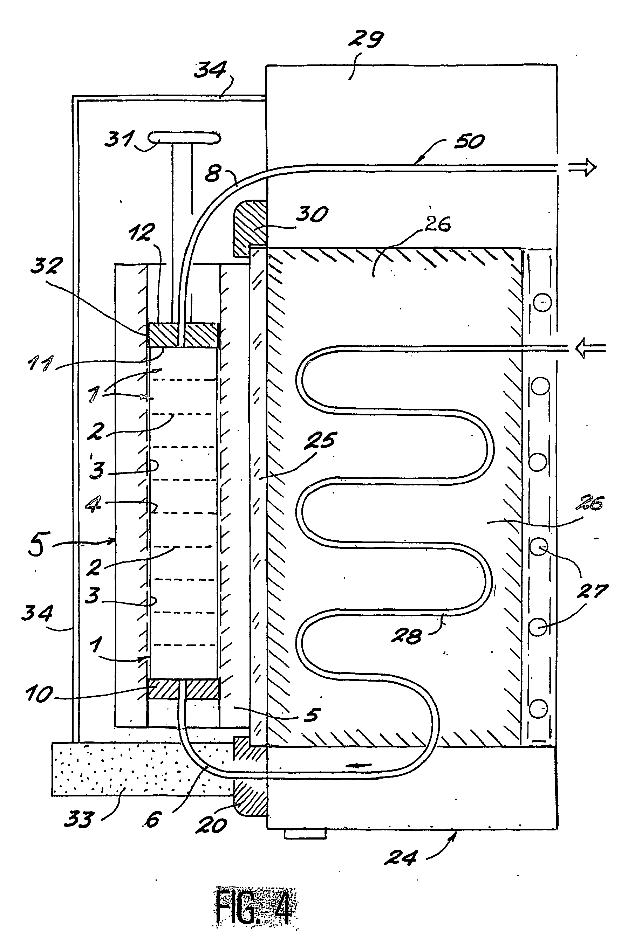

La Figure 3 illustre plus en détail quoique de façon schématique l'ensemble de l'installation.

Le canal 6 de raccordement au tube de confinement 5 est relié à une pompe électrique 13 pouvant, selon le cas, aspirer ou refouler la solution liquide qui circule à travers l'empilement des coupelles 1.

Cette pompe 13 est raccordée par une conduite 14 à une électrovanne 15, multi-voies, mettant en communication cette conduite 14 avec une tubulure de liaison telle que 16, montée entre une des sorties de cette électrovanne et un réservoir de solution 17.

Dans l'exemple de réalisation représenté, l'installation comporte neuf tubulures de liaison 16 et neuf réservoirs 17, contenant chacun une solution ou un réactif liquide différent, ces neuf réservoirs étant repérés sur la Figure par les références A à I. Neuf tubulures 16 relient ainsi la pompe 15 aux neufs réservoirs 17.

Le canal d'évacuation 8, à la partie supérieure du tube de confinement 5, est relié à une seconde électrovanne multi-voies 18, permettant dans l'exemple illustré, de sélectionner une parmi trois positions.

Dans ces conditions, cette électrovanne 18 peut relier le canal d'évacuation 8 du tube 5 par une tubulure 19 à deux réservoirs 17 donnés, ici les réservoirs F et G, afin de former avec le tube 5, le canal 6, la pompe 13 et la conduite 14, un circuit clos, en boucle fermée, désigné de façon générale sous la référence 50 et dans lequel circulent et sont recyclés la solution ou le réactif provenant du réservoir 17 ainsi sélectionné dans les deux premières positions de l'électrovanne 18.

En variante, pour la troisième position de l'electrovanne multi-voies 18, la solution provenant d'un quelconque des réservoirs 17 par sa tubulure 16 associée, après circulation dans le circuit 50 et notamment dans le tube de confinement 5, est renvoyée par une conduite 20 vers une cuve 21 d'évacuation, notamment si cette solution est impropre à une nouvelle utilisation.

La commande des positions des électrovannes 15 et 18, selon l'agencement choisi pour les tubulures 16 et 19, permet de modifier à volonté le choix des solutions contenues dans les réservoirs 17 et de la circulation de celles-ci en circuit fermé en boucle, ou ouvert avec élimination de ces solutions après utilisation lorsqu'il n'est pas nécessaire de les recycler.

Le contrôle du fonctionnement de la pompe électrique 13, notamment son sens de pompage, et celui des électrovannes multi-voies 15 et 18, est assuré par un micro-ordinateur 22, duquel partent les liaisons 23 de commande de ces appareils, les connexions électriques n'étant pas représentées pour ne pas surcharger le schéma.

Sur la Figure 3, la référence 24 désigne enfin dans son ensemble, représentée de façon très schématique, une enceinte thermostatée et thermorégulée, avantageusement prévue dans l'installation considérée et dont la réalisation apparaît plus en détail sur la Figure 4.

Le tube de confinement 5 comporte une réglette métallique latérale 25, collée ou fixée extérieurement par d'autres moyens appropriés contre une génératrice verticale de ce tube, cette réglette étant elle-même au contact d'un bloc métallique 26 sur lequel est rapportée une résistance de chauffage 27. De préférence, la réglette et le bloc sont réalisés en aluminium.

Le canal de raccordement 6 qui traverse le fond fixe 10 du tube de confinement 5 est réuni à un serpentin 28, avantageusement réalisé en acier inoxydable, rapporté et appliqué contre le bloc de métal 26 de telle sorte que la solution ou le réactif liquide qui traverse le circuit 50 soit en permanence amené à la température régulée du bloc. L'enceinte 24 est montée dans un carter extérieur de protection et d'isolation 29.

Sur la Figure 4, le fond mobile 11 à la partie supérieure du tube de confinement 5, au-dessus de l'empilement des coupelles 1, est en contact avec le piston 12, lequel se prolonge par une tige de commande 31 permettant de le manoeuvrer à l'intérieur du tube et de l'appliquer sur la coupelle supérieure. Le piston 12 comporte latéralement un racleur 32 en un matériau élastique, assurant l'étanchéité du montage et en particulier de l'empilement des coupelles 1 à l'intérieur du tube de confinement 5.

Le canal d'évacuation 8 traverse le piston 12 pour déboucher dans le tube 5 et permettre la circulation de la solution dans le circuit 50.

Le tube de confinement 1 est appliqué sur le bloc 26 et maintenu par des moyens de verrouillage 30, avantageusement disposés aux extrémités haute et basse de la réglette 25. Le tube 5 et le piston 12 sont logés dans une extension latérale 34 du carter 29, formant capot de protection, celui-ci étant fermé en partie basse par un support isolant 33.

L'installation ainsi conçue permet de réaliser, de façon automatisée et en continu, le cas échéant sous pression appropriée et à température désirée, le marquage d'échantillons biologiques en trois dimensions, avec des sondes anticorps ou enzymes ou encore par une combinaison de ces marqueurs, le micro-ordinateur 22 assurant en permanence le contrôle et la gestion des opérations réalisées, en modifiant dans chaque cas particulier la nature de la solution à utiliser, le temps de passage sur ces échantillons de cette solution, les conditions de marquage et l'enregistrement des résultats obtenus. Il permet également dé contrôler de la même façon après chaque passage de solution et/ou marquage d'un échantillon les phases éventuellement nécessaires de rinçage du circuit avant mise en oeuvre d'une nouvelle analyse.

Les exemples donnés ci-après précisent les protocoles à suivre dans les différents cas pris en considération.

### Exemple 1

Mise en évidence spécifique des ARN messagers transcrits à partir du gène Wnt-11. Ces ARN messagers sont révélateurs de l'activation du gène Wnt-11 sur des embryons de poulet de 3 et 4 jours de développement. Ce protocole permet de visualiser directement sur l'échantillon (embryon) le lieu d'activation du gène, cette activation de Wnt-11 étant révélatrice de la première étape de la formation du derme de la peau.

### - Réactifs

A - 50 ml d'éthanol destiné à placer l'échantillon dans le même solvant que celui d'un stockage opéré précédemment à -20°C (dénommé Et OH).

B - 50 ml de tampon phosphate (Phosphate Buffer Saline PBS), pH = 7,2 et 50µl de détergent Tween 20, destiné à réhydrater progressivement l'échantillon tout en conservant l'iso-tonicité et le pH physiologique nécessaire au bon fonctionnement des réactifs utilisés ultérieurement.

Le détergent est destiné à faciliter le transit des réactifs au sein de l'échantillon. (dénommé PBT).

C - 10 µl de protéinase K (à10mg/ml) dans 10 ml de réactif B. Cette enzyme est destinée à perméabiliser partiellement l'échantillon, en dégradant certaines protéines membranaires, afin d'améliorer la pénétration au sein de l'échantillon des réactifs utilisés par la suite (dénommé Pro K).

D - Fixateur 5,4 ml de formaldéhyde à 37% ; 200µl EDTA (Ethylène Diamine Tétra Acétate) à 0,5M ; 50 µl de Soude à 1M, quantité suffisante pour 50 ml PBS. Le fixateur est destiné à consolider l'échantillon qui a été partiellement fragilisé par la protéinase K. (dénommé Fix).

E - 25 ml de formamide, 12,5 ml de tampon SSC 20x (Sodium Saline Citrate), 12,5 ml d'eau, 50 µl de Tween 20. Cette solution de pré-hybridation est destinée à placer l'échantillon dans les conditions idéales favorisant l'hybridation spécifique de la sonde ARN simple brin sur l'ARN messager transcrit dans les cellules de l'échantillon (dénommé Mix).

F - 5 µl de sonde (Wnt-11), constituée d'un ARN messager anti-sens simple brin, obtenu par la transcription d'un ADN double brin codant pour la protéine Wnt-11. Cet ADN est lui-même obtenu au préalable par recombinaison moléculaire et amplification au sein d'un plasmide bactérien. L'ARN messager anti-sens est marqué au cours de sa synthèse à l'aide de nucléotides couplés chimiquement à la digoxigénine DIG. La digoxigénine joue ici le rôle de l'haptène, ou peptide pouvant être reconnu et lié spécifiquement par un anticorps; 5 ml de réactif E (dénommé Sonde).

G - 4 ml de réactif B, 1 ml de sérum, 5 µl d'anticorps monoclonal d'origine murine, dirigé spécifiquement contre la digoxigénine (DIG) et couplé à l'enzyme phosphatase alcaline, commercialisé par la société Roche (dénommé Ac).

H - Tampon NTMT, destiné à alcaliniser le milieu et composé de 1 ml NaCl 5M, 2,5 ml MgCl2 1M, 2,5 ml Tris HCl 2M pH 9,5, 50 µl de détergent Tween 20 en quantité suffisante pour 50 ml d'eau.

I - Réactif NBT/BCIP, destiné à révéler l'activité enzymatique phosphatase alcaline en produisant une coloration violette insoluble, 2,4 mg levamisol, 1 comprimé NBT/BCIP, 10 ml d'eau.

### - Programme

Les réactifs sont injectés dans le système, avec un débit continu de 1 ml par minute, selon la séquence suivante :
A+B : 15 mn, gradient continu de solution aqueuse de PBT (réhydratation)
B : 10 mn, lavage par tampon PBT
C : 7 mn, perméabilisation par la protéinase K.
B : 5 mn , lavage par tampon PBT
D: 15 mn, fixation des échantillons par la formaldéhyde
B : 5 mn, lavage par tampon PBT
E : 15 mn, pré-hybridation par la solution de formamide et SSC; montée progressive en température jusqu'à 70 °C.
F : 360 mn, hybridation par la sonde ARN anti-sens, Wnt-11 (recyclage en continu) à 70 °C.
E : 15 mn, lavage par la solution de formamide et SSC et descente en température progressive jusqu'à l'ambiante.
B 45 mn, lavage par tampon PBT
G : 360 mn, complexation de l'anticorps anti DIG (recyclage en continu)
B : 120 mn, lavage par tampon PBT
H : 10 mn, équilibrage des échantillons par le tampon pH = 9,5
I : 120 mn, révélation par le substrat NTBT/BCIP .

### - Résultats

Seules les zones de l'embryon ou le gène Wnt-11 est activé (domaines d'expression) sont colorées en violet. Dans ce cas précis, ces zones correspondent à la lèvre média dorsale du dermomyotome des somites. La coloration obtenue définit une zone en pointillé, située de part et d'autre du tube neural, révélant les cellules précurseur, à l'origine de la formation du derme et qui après divisions successives, vont coloniser l'embryon pour participer à la formation de la peau.

### Exemple 2

Mise en évidence spécifique des ARN messagers transcrits à partir du gène Msx-1. Cet exemple est similaire à l'exemple 1 ; cependant le protocole est simplifié en ceci que la première étape de réhydratation des échantillons est déjà effectuée. La dernière étape de révélation est réalisée hors de l'appareil afin que l'expérimentateur puisse arrêter la réaction au moment voulu, en fonction de l'intensité de la coloration désirée.

La sonde ARN anti-sens Msx-1 révèle l'activation du gène Msx-1 sur des embryons de poulet de 3 et 4 jours de développement. Ce protocole permet de visualiser directement sur l'échantillon (embryon) le lieu d'activation du gène, cette activation de Msx-1 étant révélatrice de la formation de la peau et des bourgeons de membres.

### - Réactifs

B - 500 ml de tampon phosphate (Phosphate Buffer Saline PBS) pH = 7,2 et 500µl détergent Tween 20, destiné à conserver l'iso-tonicité et le pH physiologique nécessaire au bon fonctionnement des réactifs utilisés ultérieurement. Le détergent est destiné à faciliter le transit des réactifs au sein de l'échantillon (dénommé PBT).

C - 60 µl protéinase K (à 10 mg/ml) dans 30 ml de réactif B. Cette enzyme est destinée à perméabiliser partiellement l'échantillon en dégradant certaines protéines membranaires, afin d'améliorer la pénétration au sein de l'échantillon des réactifs utilisés par la suite (dénommé Pro K).

D - Fixateur 5,4 ml de formaldéhyde à 37%, 200 µl EDTA (Ethylène Diamine Tétra Acétate) à 0,5M, 50 µl de Soude à 1M, quantité suffisante pour 50 ml PBS. Le fixateur est destiné à consolider l'échantillon qui a été partiellement fragilisé par la protéinase K (dénommé Fix).

E - 25 ml de formamide, 12,5 ml de tampon SSC 20x (Sodium Saline Citrate), 12,5 ml d'eau, 50 µl de Tween 20. Cette solution de pré-hybridation est destinée à placer l'échantillon dans les conditions idéales favorisant l'hybridation spécifique de la sonde ARN simple brin sur l'ARN messager transcrit dans les cellules de l'échantillon (dénommé Mix).

F - 5 µl de sonde (Msx-1) constituée d'un ARN messager anti-sens simple brin, obtenu par la transcription d'un ADN double brin codant pour la protéine Msx-1. Cet ADN est lui-même obtenu au préalable par recombinaison moléculaire et amplification au sein d'un plasmide bactérien. L'ARN messager anti-sens est marqué au cours de sa synthèse à l'aide de nucléotides couplés chimiquement à la digoxigénine DIG. La digoxigénine joue ici le rôle de l'haptène, ou peptide pouvant être reconnu et lié spécifiquement par un anticorps; 5 ml de réactif E (dénommé Sonde).

G - 4 ml de réactif B, 1 ml de sérum, 5 µl d'anticorps monoclonal d'origine murine dirigé spécifiquement contre la digoxigénine (DIG) et couplé à l'enzyme phosphatase alcaline, commercialisé par la société Roche (dénommé Ac).

H - Tampon NTMT destiné à alcaliniser le milieu et composé de 1 ml NaCl 5M, 2,5 ml MgC12 1M, 2,5 ml Tris HCl 2M pH 9,5, 50 µl de détergent Tween 20 en quantité suffisante pour 50 ml d'eau.

### - Programme

Les réactifs sont injectés dans le système, à un débit continu de 1.5 ml par minute, selon la séquence suivante :
B : 10 mn, lavage par tampon PBT
C : 12 mn, perméabilisation par la protéinase K.
B : 5 mn , lavage par tampon PBT
D: 15 mn, fixation des échantillons par la formaldéhyde
B : 5 mn , lavage par tampon PBT
E : 20 mn , pré-hybridation par la solution de formamide et SSC; montée progressive en température jusqu'à 70 °C.
F : 8 heures, hybridation par la sonde ARN anti-sens, Wnt-11 (recyclage en continu) à 70°C.
E : 20 mn , lavage par la solution de formamide et SSC; descente progressive en température jusqu'à l'ambiante.
B : 45 mn , lavage par tampon PBT
G : 7 heures, complexation de l'anticorps anti DIG (recyclage en continu).
B : 2 heures, lavage par tampon PBT
H : 10 mn, équilibrage des échantillons par le tampon pH = 9,5

### - Résultats

Seules les zones de l'embryon ou le gène Msx-1 est activé (domaines d'expression) sont colorées en violet. Dans ce cas précis, ces zones correspondent à la partie dorsale du tube neural ainsi qu'à l'AER (apical ectodermal ridge) des bourgeons de membres.

### Exemple 3

Mise en évidence spécifique des protéines Myo-D, présentes dans les cellules précurseur du muscle. Cet exemple repose sur une révélation exclusivement immunologique de la présence d'une protéine (Myo-D) à l'aide d'anticorps spécifique.

De façon résumée, l'anticorps monoclonal d'origine murine dirigé contre la protéine Myo-D est ensuite lui-même reconnu et lié à un anticorps secondaire (anti immunoglobuline de souris) lui-même marque par une liaison covalente à une enzyme : la peroxydase.

### - Réactifs

B - 700 ml de tampon phosphate (Phosphate Buffer Saline PBS) pH = 7,2 (700µl détergent Tween 20), destiné à laver l'échantillon tout en conservant l'iso-tonicité et le pH physiologique nécessaire au bon fonctionnement des réactifs utilisés ultérieurement. Le détergent est destiné à faciliter le lavage et le transfert des réactifs au sein de l'échantillon (dénommé PBT).

F - 5 µl d'anticorps monoclonal dirigé spécifiquement contre la protéine Myo-D dilués dans 10 ml de réactif B (dénommé anticorps primaire).

G - 5 µl d'anticorps polyclonal dirigé spécifiquement contre l'immunoglobuline de souris marqué par conjugaison à une enzyme, la peroxydase.

L'anticorps est dilué dans 10 ml de réactif B (dénommé anticorps secondaire).

### - Programme

Les réactifs sont injectés dans le système, à un débit continu de 1,5 ml par minute, selon la séquence définie comme suit:
B : 30 mn, équilibrage des échantillons aux conditions physiologiques
F : 8 heures 30 mn, complexation de l'anticorps primaire en recyclage continu.
B : 2 heures, lavage des échantillons afin d'éliminer les molécules liées de façon non spécifique.
G : 8 heures, complexation de l'anticorps secondaire en recyclage continu.
B : 3 heures, lavage des échantillons afin d'éliminer les molécules liées de façon non spécifique.
B : lavage en recyclage continu

Révélation de l'activité enzymatique par la diamino benzidine (DAB).

### - Résultats

Seules les zones de l'embryon ou la protéine Myo-D est exprimée (c'est à dire présente) sont colorées en brun. Dans ce cas précis, ces zones correspondent au dermomyotome des somites ainsi qu'à toutes les zones ou les muscles se mettent en place dans l'embryon à ce stade.

### Exemple 4

L'automatisation de l'appareil permet de réaliser des tâches telles que la purge des tubes afin de les remplir avec les différents réactifs utilisés. Par exemple, en reprenant l'exemple 1.

### - Réactifs

A - 50 ml d'éthanol destiné à placer l'échantillon dans le même solvant que celui du stockage opéré précédemment à -20°C (dénommé Et OH)

B - 50 ml de tampon phosphate (Phosphate Buffer Saline PBS) pH = 7,2 et 50 µl détergent Tween 20 destiné à réhydrater progressivement l'échantillon tout en conservant l'iso-tonicité et le pH physiologique nécessaire au bon fonctionnement des réactifs utilisés ultérieurement. Le détergent est destiné à faciliter le transit des réactifs au sein de l'échantillon (dénommé PBT).

C - 10 µl de protéinase K (à 10 mg/ml) dans 10 ml de réactif B. Cette enzyme est destinée à perméabiliser partiellement l'échantillon en dégradant certaines protéines membranaires, ceci afin d'améliorer la pénétration au sein de l'échantillon des réactifs utilisés ultérieurement (dénommé Pro K).

D - Fixateur 5,4 ml de formaldéhyde à 37% ; 200 µl EDTA (Ethylène Diamine Tétra Acétate) à 0,5M, 50 µl Soude à 1M, quantité suffisante pour 50 ml PBS. Le fixateur est destiné à consolider l'échantillon qui a été partiellement fragilisé par la protéinase K (dénommé Fix).

E - 25 ml de formamide, 12, 5 ml de tampon SSC 20x (Sodium Saline Citrate), 12,5 ml d'eau, 50 µl de Tween 20. Cette solution de pré-hybridation est destinée à placer l'échantillon dans les conditions idéales favorisant l'hybridation spécifique de la sonde ARN simple brin sur l'ARN messager transcrit dans les cellules de l'échantillon (dénommé Mix).

F - 5 µl de sonde (Wnt-11) constituée d'un ARN messager anti-sens simple brin, obtenu par la transcription d'un ADN double brin codant pour la protéine Wnt-11. Cet ADN est lui-même obtenu au préalable par recombinaison moléculaire et amplification au sein d'un plasmide bactérien. L'ARN messager anti-sens est marqué au cours de sa synthèse à l'aide de nucléotides couplés chimiquement à la digoxigénine DIG. La digoxigénine joue ici le rôle de l'haptène, ou peptide pouvant être reconnu et lié spécifiquement par un anticorps, 5 ml de réactif E (dénommé Sonde).

G - 4 ml de réactif B, 1 ml de sérum, 5 µl d'anticorps monoclonal d'origine murine dirigé spécifiquement contre la digoxigénine (DIG) et couplé à l'enzyme phosphatase alcaline, commercialisé par la société Roche (dénommé Ac).

H - Tampon NTMT destiné à alcaliniser le milieu et composé de 1 ml NaCl 5M, 2,5 ml MgC12 1M, 2,5 ml Tris HCl 2M pH 9,5, 50 µl de détergent Tween 20 en quantité suffisante pour 50 ml d'eau.

I - Réactif NBT/BCIP destiné à révéler l'activité enzymatique phosphatase alcaline en produisant une coloration violette insoluble 2,4 mg levamisol, 1 comprimé de NBT/BCIP, 10 ml d'eau.

### - Programme

Les réactifs sont injectés successivement dans le système, à un débit continu de 1 ml par minute, à raison de 15 secondes pour chaque réactif.

### - Résultats

Ce délai est nécessaire et suffisant pour emplir les tubulures (volume mort) correspondant à chaque réactif. De ce fait le système est prêt pour un programme d'analyse sans risquer d'injecter de l'air ou de l'eau d'un rinçage précédent.

### Exemple 5

L'automatisation de l'appareil permet de réaliser simplement le rinçage complet de l'installation en fin d'expérimentation. Pour cela les récipients contenant les réactifs sont remplis d'eau tandis qu'un programme spécifique effectue alors ce rinçage.

### - Programme

L'eau est injectée successivement dans le circuit, avec un débit continu de 1,5 ml par mn, à raison de 10 mn pour chaque tubulure d'amenée d'un réactif.

### - Résultats

Ce délai est nécessaire mais suffisant pour rincer soigneusement les tubulures, les conduites et les autres accessoires de l'installation où chaque réactif a circulé. Après quoi, l'ensemble est prêt pour un nouveau programme d'analyse.

On réalise ainsi une installation de détection modulable et automatisée des effets d'un marquage sur des molécules d'origine biologique, de conception très simple et peu coûteuse, facile à mettre en oeuvre et présentant une grande efficacité, notamment pour le traitement d'échantillons en trois dimensions.

En particulier, cette installation avec le tube de confinement contenant les coupelles, et le circuit en boucle pour la circulation des solutions et réactifs liquides, peut être miniaturisée et rendue relativement compacte, permettant par l'enceinte thermostatée et thermorégulée un contrôle précis de la température de fonctionnement. L'usage d'un micro-ordinateur de contrôle et de commande pour piloter le fonctionnement peut être réalisé sous un environnement classique du type « *Windows »* (Marque déposée), permettant de créer ou de modifier les programmes d'analyse et de les conserver en mémoire.

Bien entendu, il va cependant de soi que l'invention ne se limite pas au mode de réalisation plus spécialement décrit et schématiquement représenté sur les dessins annexés ; elle en embrasse au contraire toutes les variantes.

## Revendications

1. Installation automatisée pour la détection de la fixation de molécules d'origine biologique ou biomolécules sur un échantillon en trois dimensions, **caractérisée en ce qu'**elle comporte:
- une pluralité de coupelles individuelles (1) de réception d'un ou plusieurs échantillons à traiter, comportant chacune une paroi latérale (3) fermée par un fond perforé (2) pour le passage en continu à l'intérieur de la coupelle de solutions ou réactifs liquides à mettre en contact avec l'échantillon, ces coupelles présentant le même encombrement diamétral et étant logées en superposition verticale dans un tube (5) ou une enveloppe externe analogue de positionnement et de confinement,
- un circuit (50) pour l'écoulement en continu à travers les coupelles (1) dans le tube de confinement (5), sous pression réglable, de ces solutions, provenant d'autant de réservoirs distincts (17), ces solutions étant introduites à la demande, ensemble ou séparément dans ce circuit,
- une électrovanne multi-voies (15), propre à relier séparément chaque réservoir (17) de solutions au circuit (50),
- une pompe électrique (13), de préférence à inversion du sens de pompage et de refoulement, apte à faire circuler en continu dans le circuit (50) les quantités de solutions prélevées depuis les réservoirs,
- et un moyen de contrôle et de commande séquentiel et coordonné (22) de la pompe (13) et de l'électrovanne multi-voies (15).

2. Installation selon la revendication 1, **caractérisée en ce que** le circuit (50) est un circuit fermé, en boucle pour le recyclage permanent des solutions.

3. Installation selon la revendication 1, **caractérisée en ce que** le circuit (50) est un circuit ouvert pour l'élimination des solutions après au moins un passage à travers les coupelles (1) dans le tube de confinement (5).

4. Installation selon l'une quelconque des revendications 1 à 3, **caractérisée en ce que** les coupelles (1) sont empilées dans le tube de confinement (5) disposé verticalement, l'écoulement des solutions dans le tube s'effectuant dans le sens ascendant.

5. Installation selon la revendication 1, **caractérisée en ce qu'**elle comporte une seconde électrovanne multi-voies (18) permettant, soit la récupération de certaines au moins des solutions pour les faire circuler en boucle dans le circuit (50), soit l'élimination de ces solutions la récupération de certaines au moins des solutions ayant circulé dans le circuit en boucle (50), renvoyées dans leurs réservoirs (17) et éliminées depuis ceux-ci vers un récipient d'évacuation extérieur (21).

6. Installation selon l'une des revendications 1 à 5, **caractérisée en ce que** les coupelles (1) de réception des échantillons sont cylindriques et présentent la même hauteur d'une coupelle à l'autre.

7. Installation selon l'une des revendications 1 à 5, **caractérisée en ce que** les coupelles (1) présentent des hauteurs différentes.

8. Installation selon l'une quelconque des revendications 1 à 4, **caractérisée en ce que** les coupelles (1) de réception des échantillons, empilées dans le tube de confinement (5), sont immobilisées entre un fond inférieur fixe (10), fermant ce tube, et un fond supérieur mobile (11), dont le déplacement à l'intérieur du tube pour s'appliquer sur l'empilement des coupelles, est commandé par un piston d'actionnement (12) extérieur à ce tube, plongeant dans ce dernier et comportant un canal axial (8) pour la circulation des solutions dans le circuit(50).

9. Installation selon la revendication 8, **caractérisée en ce que** le fond mobile (11) est équipé d'un racleur étanche (32), en un matériau élastique approprié, portant contre la surface interne du tube de confinement (5).

10. Installation selon l'une quelconque des revendications 1 à 9, **caractérisée en ce que** la paroi latérale (3) de chaque coupelle (1) est transparente et réalisée en un matériau plastique, notamment en un polymère tel que du polystyrène, du métacrylate de méthyle ou du polycarbonate.

11. Installation selon l'une quelconque des revendications 1 à 10, **caractérisée en ce que** le fond (2) de chaque coupelle (1) est constitué par un tamis à mailles fines, notamment en toile de polyester ou de *« Nylon* ».

12. Installation selon l'une quelconque des revendications 1 à 11, **caractérisée en ce que** le tube de confinement (5) est réalisé en verre ou en une matière plastique transparente.

13. Installation selon l'une quelconque des revendications 1 à 12, **caractérisée en ce que** les moyens de contrôle et de commande séquentiels (22) comportent un micro-ordinateur qui détermine la composition, l'ordre de leur écoulement dans le circuit et la durée de mise en contact des solutions ou réactifs avec les échantillons dans les coupelles (1), ainsi que le débit de l'écoulement de ces solutions.

14. Installation selon l'une quelconque des revendications 1 à 13, **caractérisée en ce que** le tube de confinement (5) est logé dans une enceinte (24) thermostatée et thermorégulée.

15. Installation selon la revendication 14, **caractérisée en ce que** l'enceinte thermostatée (24) comporte une réglette métallique (25), collée ou autrement appliquée contre une génératrice du tube de confinement (5), cette réglette étant maintenue en contact avec un bloc de métal massif (26), contre lequel est rapporté un élément (27) propre à réaliser un chauffage contrôlé de ce bloc.

16. Installation selon la revendication 15, **caractérisée en ce que** le circuit (50) comporte un serpentin (28) scellé dans le bloc métallique (26), placé en série dans ce circuit.

17. Installation selon la revendication 16, **caractérisée en ce que** le serpentin (28) est réalisé en acier inoxydable et le bloc métallique (26) en aluminium.

18. Installation selon l'une quelconque des revendications 15 à 17, **caractérisée en ce que** le tube de confinement (5) et la réglette métallique (25) sont entourés par un capot de protection (34), de préférence en matière plastique.

## Claims

1. Automated installation for detecting the fixation of molecules of biological origin or biomolecules on a sample in three-dimensions, **characterized in that** it comprises:
- a plurality of individual dishes (1) for receiving one or more samples to be treated, each comprising a side wall (3) closed by a perforated base (2) for the continuous passage inside the dish of solutions or liquid reagents to be brought into contact with the sample, these dishes having the same diametral space requirement and being accommodated in vertical superposition in a tube (5) or a similar external positioning and confining casing,
- a circuit (50) for the continuous flow through the dishes (1) into the confining tube (5), under adjustable pressure, of these solutions, originating from distinct reservoirs (17), these solutions being introduced on demand, together or separately into this circuit,
- a multipath solenoid valve (15), suitable for separately connecting each solution reservoir (17) with the circuit (50),
- an electric pump (13), preferably reversing the pumping and backflow direction, capable of effecting the continuous circulation in the circuit (50) of the quantities of solutions taken from the reservoirs,
- and a sequential and coordinated control and command means (22) of the pump (13) and of the multipath solenoid valve (15).

2. Installation according to claim 1, **characterized in that** the circuit (50) is a closed loop circuit, for the permanent recycling of the solutions.

3. Installation according to claim 1, **characterized in that** the circuit (50) is an open circuit for the removal of solutions after at least one passage through the dishes (1) in the confining tube (5).

4. Installation according to any one of claims 1 to 3, **characterized in that** the dishes (1) are stacked in the confining tube (5) arranged vertically, with the solutions flowing upwards in the tube.

5. Installation according to claim 1, **characterized in that** it comprises a second multipath solenoid valve (18) allowing, either the recovery of at least some of the solutions in order to cause them to circulate in a loop in the circuit (50), or the removal of these solutions the recovery of at least some of the solutions having circulated in the loop circuit (50), returned to their reservoirs (17) and removed from the latter to an external evacuation receptacle (21).

6. Installation according to one of claims 1 to 5, **characterized in that** the dishes (1) for receiving the samples are cylindrical and have the same height from one dish to another.

7. Installation according to one of claims 1 to 5, **characterized in that** the dishes (1) have different heights.

8. Installation according to any one of claims 1 to 4, **characterized in that** the dishes (1) for receiving the samples, stacked in the confining tube (5), are immobilized between a fixed lower base (10), closing this tube, and a mobile upper base (11), the displacement of which inside the tube in order to be applied to the stack of dishes, is controlled by an actuation piston (12) outside this tube, plunging into the latter and comprising an axial channel (8) for the circulation of the solutions in the circuit (50).

9. Installation according to claim 8, **characterized in that** the mobile base (11) is equipped with a tight scraper (32), made of an appropriate elastic material, bearing against the internal surface of the confining tube (5).

10. Installation according to any one of claims 1 to 9, **characterized in that** the side wall (3) of each dish (1) is transparent and made of a plastic material, in particular a polymer such as polystyrene, methyl methacrylate or polycarbonate.

11. Installation according to any one of claims 1 to 10, **characterized in that** the base (2) of each dish (1) is constituted by a fine-meshed sieve, in particular made of polyester or *"Nylon"* cloth.

12. Installation according to any one of claims 1 to 11, **characterized in that** the confining tube (5) is made of glass or a transparent plastic material.

13. Installation according to any one of claims 1 to 12, **characterized in that** the sequential control and command means (22) comprise a micro-computer which determines the composition, the order of their flow in the circuit and the period for which the solutions or reagents are brought into contact with the samples in the dishes (1), as well as the flow rate of these solutions.

14. Installation according to any one of claims 1 to 13, **characterized in that** the confining tube (5) is accommodated in a thermostatically controlled and thermoregulated enclosure (24).

15. Installation according to claim 14, **characterized in that** the thermostatically controlled enclosure (24) comprises a metallic strip (25), glued or otherwise applied to a generatrix of the confining tube (5), this strip being maintained in contact with a block of solid metal (26), against which is mounted an element (27) suitable for achieving controlled heating of this block.

16. Installation according to claim 15, **characterized in that** the circuit (50) comprises a coil (28) sealed in the metal block (26), placed in series in this circuit.

17. Installation according to claim 16, **characterized in that** the coil (28) is made of stainless steel and the metal block (26) of aluminium.

18. Installation according to any one of claims 15 to 17, **characterized in that** the confining tube (5) and the metallic strip (25) are surrounded by a protective cover (34), preferably made of plastic.

## Patentansprüche

1. Automatisierte Anlage für die Detektion der Fixierung von Molekülen biologischen Ursprungs oder Biomolekülen auf einer Probe in drei Dimensionen, **dadurch gekennzeichnet, dass** sie aufweist:
- eine Vielzahl einzelner Schalen (1) für die Aufnahme einer oder mehrerer zu behandelnder Proben, wobei jede Schale eine Seitenwand (3) aufweist, abgeschlossen durch einen perforierten Boden (2) für den kontinuierlichen Durchgang von flüssigen Lösungen oder Reagenzien, die mit der Probe in Kontakt gebracht werden sollen, in das Innere der Schale, wobei die Schalen den gleichen Durchmesser aufweisen und vertikal übereinander in einem Rohr (5) oder einer äußeren Hülle mit analoger Positionierung und Umschließung angeordnet sind,
- einen Kreislauf (50) für den kontinuierlichen Durchfluss der von ebenso vielen verschiedenen Behältern (17) kommenden Lösungen durch die Schalen (1) in dem Umschließungsrohr (5) unter regelbarem Druck, wobei diese Lösungen nach Bedarf gemeinsam oder getrennt in diesen Kreislauf eingeführt werden,
- ein Mehrweg-Elektroventil (15), das geeignet ist, jeden Behälter (17) von Lösungen separat mit dem Kreislauf (50) zu verbinden,
- eine Elektropumpe (13), vorzugsweise mit Umkehrung der Pumprichtung und Förderung, um die Lösungsmengen, die aus den Behältern entnommen werden, kontinuierlich in dem Kreislauf (50) zirkulieren zu lassen,
- und ein Mittel (22) zur aufeinanderfolgenden und koordinierten Regelung und Steuerung der Pumpe (13) und des Mehrweg-Elektroventils (15).

2. Anlage nach Anspruch 1, **dadurch gekennzeichnet, dass** der Kreislauf (50) ein geschlossener Kreislauf ist, in einer Schleife für das permanente Recycling von Lösungen.

3. Anlage nach Anspruch 1, **dadurch gekennzeichnet, dass** der Kreislauf (50) ein offener Kreislauf ist, zum Entfernen von Lösungen nach mindestens einem Durchgang durch die Schalen (1) in dem Umschließungsrohr (5).

4. Anlage nach einem der Ansprüche 1 bis 3, **dadurch gekennzeichnet, dass** die Schalen (1) in dem vertikal angeordneten Umschließungsrohr (5) gestapelt sind, wobei das Hindurchströmen der Lösungen in dem Rohr aufsteigend erfolgt.

5. Anlage nach Anspruch 1, **dadurch gekennzeichnet, dass** sie ein zweites Mehrweg-Elektroventil (18) aufweist, das erlaubt, dass zumindest einige der Lösungen zurückgewonnen werden, um sie.in der Schleife im Kreislauf (50) zirkulieren zu lassen, oder dass diese Lösungen entfernt werden, deren Wiedergewinnung von zumindest bestimmten Lösungen in dem schleifenförmigen Kreislauf (50) zirkuliert wurde, die in ihre Behälter (17) zurückgeführt wurden und die von diesen zu einem Entsorgungsbehälter (21) abgeführt werden.

6. Anlage nach einem der Ansprüche 1 bis 5, **dadurch gekennzeichnet, dass** die Schalen (1) für die Aufnahme der Proben zylindrisch sind und von einer Schale zur anderen die gleiche Höhe aufweisen.

7. Anlage nach einem der Ansprüche 1 bis 5, **dadurch gekennzeichnet, dass** die Schalen (1) verschiedene Höhen aufweisen.

8. Anlage nach einem der Ansprüche 1 bis 4, **dadurch gekennzeichnet, dass** die in dem Umschließungsrohr (5) gestapelten Schalen (1) für die Aufnahme der Proben zwischen einem fixen unteren Boden (10), der das Rohr abschließt, und einem beweglichen oberen Boden (11), dessen Verschiebung ins Innere des Rohres zum Aufbringen auf den Schalenstapel durch einen Betätigungskolben (12) außerhalb des Rohres erfolgt, der in letzteres eintaucht und einen axialen Kanal (8) für das Zirkulieren von Lösungen in dem Kreislauf (50) aufweist, immobilisiert sind.

9. Anlage nach Anspruch 8, **dadurch gekennzeichnet, dass** der bewegliche Boden (11) mit einem dichtenden Schieber (32) aus einem geeigneten elastischen Material versehen ist, der an der Innenfläche des Umschließungsrohres (5) anliegt.

10. Anlage nach einem der Ansprüche 1 bis 9, **dadurch gekennzeichnet, dass** die Seitenwand (3) jeder Schale (1) transparent ist und aus einem Kunststoffmaterial ausgeführt ist, insbesondere einem Polymer wie Polystyrol, Methylmethacrylat oder Polycarbonat.

11. Anlage nach einem der Ansprüche 1 bis 10, **dadurch gekennzeichnet, dass** der Boden (2) jeder Schale (1) aus einem Sieb mit feinen Löchern besteht, insbesondere aus Polyester- oder Nylonstoff.

12. Anlage nach einem der Ansprüche 1 bis 11, **dadurch gekennzeichnet, dass** das Umschließungsrohr (5) aus Glas oder einem transparenten Kunststoffmaterial ausgeführt ist.

13. Anlage nach einem der Ansprüche 1 bis 12, **dadurch gekennzeichnet, dass** die aufeinanderfolgenden Mittel (22) zur Regelung und Steuerung einen Mikrocomputer umfassen, der die Zusammensetzung, die Reihenfolge des Durchflusses in dem Kreislauf und die Dauer des Kontaktes der Lösungen oder Reagenzien mit den Proben in den Schalen (1) sowie die Durchflussmenge der Lösungen bestimmt.

14. Anlage nach einem der Ansprüche 1 bis 13, **dadurch gekennzeichnet, dass** das Umschließungsrohr (5) in einer thermostabilisierten und wärmegeregelten Ummantelung (24) angeordnet ist.

15. Anlage nach Anspruch 14, **dadurch gekennzeichnet, dass** die thermostabilisierte Ummantelung (24) eine Metallleiste (25) aufweist, die auf eine Mantellinie des Umschließungsrohres (5) geklebt oder auf andere Weise daran angebracht ist, wobei diese Leiste mit einem massiven Metallblock (26) in Kontakt gehalten wird, an den ein Element (27) angefügt ist, das zur Durchführung einer geregelten Erwärmung des Blocks geeignet ist.

16. Anlage nach Anspruch 15, **dadurch gekennzeichnet, dass** der Kreislauf (50) eine Rohrschlange (28) beinhaltet, die in dem Metallblock (26) eingeschlossen ist, in Serie in dem Kreislauf angeordnet.

17. Anlage nach Anspruch 16, **dadurch gekennzeichnet, dass** die Rohrschlange (28) aus Edelstahl und der Metallblock (26) aus Aluminium ausgeführt ist.

18. Anlage nach einem der Ansprüche 15 bis 17, **dadurch gekennzeichnet, dass** das Umschließungsrolu (5) und die Metallleiste (25) von einer Schutzhaube (34) umgeben sind, vorzugsweise aus einem Kunststoffmaterial.
